# EUROPEAN PATENT SPECIFICATION

(11) **EP 4 090 294 B1**
(45) Date of publication and mention of the grant of the patent: **29.04.2026**
(21) Application number: 21702861.2
(22) Date of filing: 14.01.2021
(51) Int. Cl.: A61F 2/52

(54) **AN EXTERNAL BREAST PROSTHESIS**
EXTERNE BRUSTPROTHESE
PROTHÈSE MAMMAIRE EXTERNE

(30) Priority: 17.01.2020 NL 2024688
(43) Date of publication of application: 23.11.2022
(73) Proprietor: van der Horst, Joseph, 6051 NC Maasbracht (NL); Schlösser, Monica Hubertina Johanna, 6077 GC Sint Odiliënberg (NL); Reutelingsperger, Christiaan M. H. G., 5944 BK Arcen (NL)
(72) Inventor: SCHLÖSSER, Monica Hubertina Johanna, 6077 GC Sint Odiliënberg (NL); REUTELINGSPERGER, Christiaan Mathias Hubertus Gerard, 5944 BK Arcen (NL)
(74) Representative: Janssen, Paulus J. P.
(86) International application number: PCT/EP2021/050655
(87) International publication number: WO 2021/144346

(56) References cited:
- WO-A1-2013/091720
- JP-A- H05 131 007
- US-A1- 2012 010 705
- US-A1- 2014 309 750
- ZHU BOWEN ET AL: "Novel Polyethylene Fibers of Very High Thermal Conductivity Enabled by Amorphous Restructuring", ACS OMEGA, vol. 2, no. 7, 26 July 2017 (2017-07-26), US, pages 3931 - 3944, XP055795048, ISSN: 2470-1343, Retrieved from the Internet <URL:http://pubs.acs.org/doi/pdf/10.1021/acsomega.7b00563> [retrieved on 20210414], DOI: 10.1021/acsomega.7b00563

## Description

### GENERAL FIELD OF THE INVENTION

The invention in general pertains to an external breast prosthesis comprising a main body made from a polymer, the body having an inner surface for coinciding with an outer chest surface of a person wearing the breast prosthesis and an opposing outer surface. The prosthesis according to the invention allows for a convenient accommodation for an anomalous breast shape of a person due to surgery or other event inducing a shape differentiation, or a naturally existing shape differentiation.

### BACKGROUND OF THE INVENTION

A shape differentiation in breast typically occurs after surgery for breast cancer. During such surgery typically a certain amount of breast tissue is be removed from a patient. Even a small amount of tissue removed creates a deficit that can't be overlooked, and causes anxiety. For example, it may become difficult to obtain satisfactory undergarments that will compensate for the deficit. In addition, existing brassieres may create pressure from the brassiere structure, especially the chest/back band. This pressure is often a source of pain at the breast surgery site in many users, which may become very fatiguing and taxing as a day wears on. Straps and bands, while managing weight, volume and position, cause significant discomfort and often, pain, especially in post-surgical situations. In practice, the deficit is often balance using an external breast prosthesis, the use of which might also decrease discomfort at the surgery site. The prosthesis typically is a structure of a material that resembles the look and/or feel of an intact natural breast, which structure may be combined with other materials such as a liner.

There are various types of traditional external post-mastectomy and lumpectomy prostheses, also called breast forms. The type of prosthesis required is determined by the amount of breast tissue that is removed. A prosthesis can be worn against the skin, inside the pocket of a mastectomy bra, or attached to the chest wall. Prosthetic devices are designed to look feminine while ensuring comfort.

The most common type of external prosthesis is the silicone breast prosthesis, such as for example known from WO2013/091720. An external silicone breast prosthesis is a weighted prosthesis, typically made of silicone polymer, which is designed to simulate natural breast tissue. The idea is that since this type of breast prosthesis is weighted, it may help the patient's posture, prevent shoulder drop, and problems with balance. An alternative type of prosthesis is a non-silicone light-weight breast form made of foam or fiberfill. Non-silicone breast prostheses are typically worn during exercise, swimming, and hot weather. However, the feel of such a light weight prosthesis is very unnatural. Another type of prosthesis is a self-adhesive breast form that attaches securely to the chest wall with adhesive strips. Self-adhesive prostheses however require regular cleaning. Yet another type of prosthesis is a soft form worn in camisole, thus providing a light-weight, removable breast form that fits into a camisole garment (a soft, stretchy garment with lace elastic straps that can be pulled up over the hips if raising the arms is difficult). Post-surgical camisole is often worn immediately following a mastectomy, lumpectomy, radiation therapy, or during reconstruction breast surgery. However, a camisole is less suitable for every day long term use.

In practice, the most used type of external breast prosthesis is the silicone breast prosthesis, mainly because of the most natural look and feel. In general, polymer type prostheses are better able to mimic natural breast tissue. However, many patients find these prostheses uncomfortable, cumbersome and impractical. Polymer prostheses are often described as heavy and irritating the skin, the latter mainly due to experienced heat buildup at the chest directly underneath the breast prosthesis. Partly this is due to the inherent low heat conductive capacity of common polymers from which these prostheses are made.

### OBJECT OF THE INVENTION

It is an object of the invention to provide an improved an external breast prosthesis comprising a main body made from a polymer, which prosthesis is more comfortable to wear for the patient.

### SUMMARY OF THE INVENTION

The objective is solved by the invention which is defined by the claims.

It was found that the transport of heat away from the contact surface between the chest of the patient and the polymer breast prosthesis can be vastly improved by incorporating in the prosthesis multiple elongated thermal conductors that are able to conduct heat away from the chest. By the fact that each of the multiple elongated thermal conductors extends from a position adjacent an inner surface of the breast prosthesis to a position adjacent to an outer surface of the breast prosthesis, heat built up at the inner surface can be effectively transferred to the outer surface of the breast prosthesis, mimicking transport of heat to the outer surface of the natural breast via transport of blood through veins. The invention enables individualization of the heat transport capacity for any breast prosthesis. It is highly likely that heat production is not entirely even across the surface of the chest part that coincides with the prosthesis. In particular, due to the surgery, particular local sites of this surface may give rise to substantially increased heat production for example due to the presence of particular tissue or an increased density of veins as a result of the surgery. This can be easily established by using an IR camera to asses local heat production. The invention enables to increase heat transport locally, for example by providing more or thicker conductors at a site where there is an increase heat production. Also, even when heat production would be the same across the chest surface, which is highly unlikely, a patient may experience an uneven temperature due to other reasons such as different heat transport due to the type of clothing, different type of exercise, or even simply due to the nerve system raising the impression of uneven temperature. This can all be accommodated by the use of multiple elongated thermal conductors in the prosthesis according to the present invention.

It is noted that that the prosthesis may be manufactured by including various other items into the prosthesis which can be used either cosmetically, for comfort, or even medically. Such items may be for example additional fillers to obtain particular (local) densities to perfectly match a natural breast in its mechanical properties, or to increase comfort, and it may even comprise sensors to sense all kinds of body parameters, or medicaments for gradual transdermal release into the body of the wearer. The latter also includes hormones that are typically used during treatment against cancer. It is also foreseen to incorporate actuators for local stimulation of body tissue of the wearer. In any case, any kind of item or compound can be incorporated into the prosthesis of the invention.

### DEFINITIONS

An *external breast prosthesis* is an artificial breast that is worn under clothing to imitate the shape of the breast. There are a wide variety of external breast prostheses available. Some are held within specially designed bras, while others are attached to the skin with a sticky backing.

The *inner surface* of an external breast prosthesis is the surface of the prosthesis that is designed for coinciding (adjoining.abutting) with an outer chest surface of a person wearing the breast prosthesis, notwithstanding that in between the chest surface and the inner surface of the prosthesis itself there is a liner, like a piece of cloth or otherwise. The inner surface of the prosthesis is opposed by an *outer surface* that provides the outer visible shape of the breast prosthesis.

*A 3D image* of a structure is a data set representing an actual three dimensional structure.

*Reticulated* means resembling a net or network, typically having veins, fibers, or lines that cross.

A *polymer* is a substance (a material) that has a molecular structure consisting substantially of macromolecules that consists of a large number of similar units bonded together, e.g., many synthetic organic materials used as plastics and resins. Small compounds are often present in the substance to enhance its properties (e.g. preservatives, UV-stabilisers, viscosity modifiers, flame retardants etc), but the amount of macromolecules is such that the overall physical properties determine that the substance has the physical properties of a polymer (also called a plastic) including its toughness, viscoelasticity, and a tendency to form glasses and semicrystalline structures rather than crystals.

A *thermoplastic elastomer* (TPE) is an elastomer comprising a thermoreversible network. A TPE belongs to a class of copolymers and/or a physical mix of polymers (usually a plastic and a rubber) that consist of materials that provide the TPE with both thermoplastic and elastomeric properties. A TPE typically has the ability to be stretched to moderate elongations and, upon the removal of stress, return to something close to its original shape, is processable as a melt at elevated temperature, and shows no significant creep. There are six generic classes of commercial TPEs: styrenic block copolymers (TPS or TPE-s), thermoplastic polyolefinelastomers (TPO or TPE-o), thermoplastic vulcanizates (TPV or TPE-v), thermoplastic polyurethanes (TPU), thermoplastic copolyester (TPC or TPE-E), thermoplastic polyamides (TPA orTPE-A). Next to this there is class of non-classified thermoplastic elastomers called TPZ. Examples of TPE materials that come from block copolymers group are amongst others CAWITON, THERMOLAST K, THERMOLAST M, Arnitel, Hytrel, Dryflex, Mediprene, Kraton, Pibiflex, Sofprene, and Laprene. Out of these styrenic block copolymers (TPE-s) are CAWITON, THERMOLAST K, THERMOLAST M, Sofprene, Dryflex and Laprene. Desmopan or Elastollan are examples of thermoplastic polyurethanes (TPU). Santoprene, Termoton, Solprene, THERMOLAST V, Vegaprene, or Forprene are examples of TPV materials. Examples of thermoplastic olefin elastomers (TPO) compound are For-Tec E or Engage.

*Additive manufacturing* (official industry standard term according to ASTM F2792) or simply "AM", is defined as the process of joining materials to make objects from a 3D image, usually layer upon layer, as opposed to subtractive manufacturing methodologies in which material is removed by machining. Common to additive manufacturing (AM) is the use of a computer, 3D modeling software (Computer Aided Design or CAD), machine equipment and layering material. Once a CAD sketch, i.e. the 3D image, is produced, the AM equipment reads in the 3D image and lays downs or adds successive parts of liquid, powder, sheet material or other, usually in a layer-upon-layer fashion to manufacture a 3D structure. The term *additive manufacturing* encompasses many technologies including subsets like 3D Printing, Rapid Prototyping (RP), Direct Digital Manufacturing (DDM), layered manufacturing and additive fabrication.

*Solid foams* are a class of lightweight cellular materials. These foams are typically classified into two types based on their pore structure: open-cell-structured foams (also known as reticulated foams) and closed-cell foams. At high enough cell resolutions, any type can be treated as continuous or "continuum" materials and are referred to as cellular solids, with predictable mechanical properties. Open-cell-structured foams contain pores that are connected to each other and form an interconnected network. Open-cell foams fill with whatever gas surrounds them. In a reticulated foam the pores are continuous, form inner wall to outer wall, as opposed to local pores. Local pores may only permit local air flow through the structure whereas continuous pores permit air flow through the complete structure, hence enabling the transport of moisture and some heat throughout the entire structure.

### FURTHER EMBODIMENTS OF THE DISCLOSURE

In a further embodiment the elongated thermal conductors are fibres, preferably polymer fibres. Fibres have the advantage that they may have little influence on the bulk properties of the polymer. Polymer conductors have found to be suitable for use in an external breast prosthesis. Although inherently being worse thermal conductors than for example (most) metal or carbon fibres, the heat conductivity of polymers may suffice for the current application, in particular when using an oriented strand polymer like DYNEEMA (DSM, Heerlen, The Netherlands). Such polymers have a very high heat conductivity and still have the mechanical properties (in particular the flexibility and density) of a polymer making them ideally suitable for incorporation as thermal conductors in a polymeric breast prosthesis, without interfering too much with the mechanical bulk properties of the polymer of which the main body of the prosthesis is made.

In another embodiment the main body consists essentially of a thermoplastic elastomer. Thermoplastic elastomers appear to be highly suitable to produce a breast prosthesis that is able to minic natural breast tissue. Although being somewhat hard in bulk, when incorporating voids in the material (5-95%), for example by providing the elastomer in the form of a foam, the prosthesis is found acceptable for the large majority of patients if not all.

In yet a further embodiment that the main body is a reticulated solid foam. It was found that the combination of providing the structure as a reticulated solid foam and at the same time making sure the polymer is a thermoplastic elastomer, the mechanical properties of a natural breast tissue can be resembled very adequately, while at the same time having a density less than common silicon polymer filled external prostheses. This means that the prosthesis may not feel "heavy" despite having adequate mechanical properties. For example, by varying the porosity (i.e. the volumetric void fraction) in various areas of the structure, the mechanical properties of varies areas in a natural breast can be mimicked very adequately. Next to this, the reticulated solid foam structure allows for any surrounding air to be able and freely penetrate completely through the prosthesis. This way, moist can be transported relatively easily from an inner surface of the prosthesis to an outer surface through the open and continuous pores, which in addition to an improved heat transport capacity may contribute significantly to a comfortable wearing experience.

Additionally, it was found that the combination of using a thermoplastic elastomer and providing the main body in the form of a reticulated solid foam enables the use of a relatively simple single-piece manufacturing technique can be used, *i.e.* a technique wherein the basic structure of the prosthesis may be formed as a single piece of material (not excluding the use of an additional material for example as a liner, a shield, protector, a dispersed filler etc.). This is as opposed to a novel technology for manufacturing breast prostheses based on so called additive manufacturing technologies as described in US 2017/0281367. In this patent it is described to produce a prosthesis to perfectly fit an individual using novel 3D methods. For this a 3D image is made of the breast prothesis, based on 3D scans of the patient in various positions, taking before and/or after the surgery. Based on the image, a unique breast prosthesis is made using selective laser sintering. The prosthesis is built up out of multiple separate parts that allow a variability enhanced over single-piece (unitary) manufacturing techniques. This leads to a prosthesis that comprises an inner wall mesh having a first density (the inner wall mesh being configured to coincide with a chest wall of the patient), and which inner wall mesh typically has fixed intersections. The breast prosthesis further comprises a distinct outer wall mesh having a second density. The outer wall mesh is configured to have an ideal shape for the patient. The outer wall mesh typically has moveable intersections to provide additional movement and flexibility. The prosthesis further comprises a band mesh having a density greater or equal to the first or second density of the inner wall mesh and outer wall mash, respectively. Lastly, the breast prosthesis comprises a central portion disposed in between the inner and outer wall meshes. The central portion may be filled with foam, batting, gel, or another suitable material. This technology wherein the prosthesis is composed out of multiple separate parts instead of using a single-piece structure, allows for the manufacture of an individualized external breast prosthesis that is comfortable to wear. However, this is at the cost of a very complicated manufacturing process to produce a multi-piece (assembled) breast prosthesis. The present disclosure thus allows the provision of an easy to manufacture external breast prosthesis, comfortable to wear and having a very good look and feel, the prosthesis comprising as a basic structure (i.e. more than 60% of the volume and/or weight of the ultimate configuration to wear, in particular at least 65%, 70, 75, 80, 81, 82, 83, 84, 85, 86, 87, 88, 89, 90, 91, 92, 93, 94, 95, 96, 97, 98, 99 or even higher) a reticulated solid foam made of a thermoplastic elastomer. This way the breast prosthesis may be a unitary structure of a reticulated solid foam, instead of a multi-piece prosthesis as known form the art.

In a further embodiment, the porosity of the reticulated solid foam, i.e. the volumetric void fraction in the solid foam, is between 50 and 95%. It was found that when using a TPE as polymer, at such a void fraction, adequate physical properties of the prosthesis for all various essential aspects (mechanical feel, heat transport, moist transport, density) can be provided in one and the same structure. Most advantageously, the porosity of the reticulated solid foam is between 60 and 85%. The porosity need not be the same throughout the volume of the prosthesis, but may locally vary to meet locally desired properties.

In yet a further embodiment, the thermoplastic elastomer is a styrene block copolymer. This particular copolymer was found to be very suitable for making a breast prosthesis with the method of the current invention. A particular suitable copolymer was found to be a styrene-ethylene-butylene-styrene copolymer. The type of polymer allows manufacturing of very comfortable prostheses, while at the same time being safe upon prolonged skin contact.

In a further embodiment, in the manufacturing process the thermoplastic elastomer is deposited to become part of the structure in the form of multiple separate droplets that fuse together after being deposited. So instead of using a technique wherein in a continuous layer of a particular material certain parts are fused to become solid, separate drops of elastomer are deposited on demand on the growing structure (corresponding to the 3D image of the structure to be formed) and fuse with the previously deposited droplets right after their deposition. This technology has shown to be particularly advantageous for manufacturing a reticulated solid foam of a thermoplastic elastomer.

In an embodiment, the manufacturing is performed using a 3D printing technology, for example an inkjet deposition technology (regarding the latter, although the polymer might not be coloured and thus as such, might not be regarded as an "ink", due to resemblance with drop-on-demand inkjet technology, this term is also used for this type of AM technologies). This technology was found to be particularly suitable for use in the present method.

The disclosure will now be further illustrated using the following particular examples.

### EXAMPLES

Figure 1 represents a flow scheme of a manufacturing method for an external breast prosthesis.
Figure 2 schematically depicts an external breast prosthesis.
Figure 3 schematically depicts a reticulated solid foam.

Example 1 describes a method of manufacturing a breast prosthesis.

Example 2 describes various tests of breast prostheses.

### Figure 1

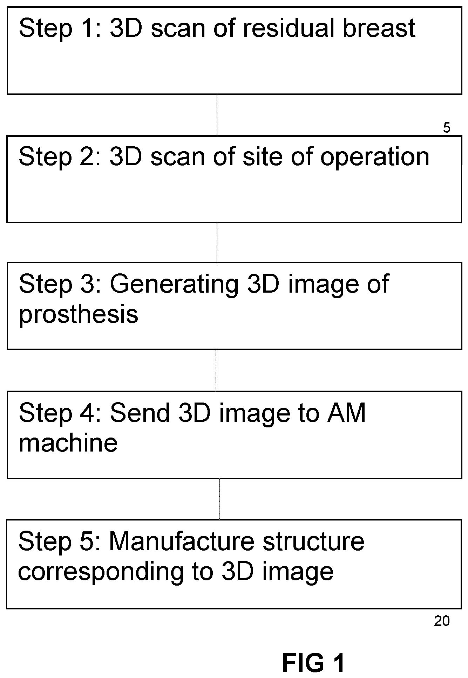

Figure 1 represents a flow scheme of a manufacturing method for an external breast prosthesis. The method aims at providing a desired shape for a breast that is partially removed during operation, the desired shape after operation being formed by the remaining breast material plus the external breast prosthesis. Step 1 of the method involves the generation of a 3D scan of the residual breast after a partial mastectomy of the diseased breast in order to generate an image of the desired shape, albeit as a mirror image which means the scanned image will be mirrored in order to correspond to the desired shape (alternatively the breast to be operated upon is scanned before the operation when the shape at this stage is the desired shape).

In Step 2 a 3D scan is made of the site of operation, thus providing an image of the chest of the patient and remaining breast tissue (if any), on to which the external prosthesis has to be placed in order to arrive at the desired shape for the operated breast.

In Step 3 a 3D image of the prosthesis is generated using the 3D scan of the residual breast (mirror imaged) and the 3D scan of the site of operation. The part of the image that misses in the latter scan when compared to the former corresponds to the external breast prosthesis.

In Step 4 this 3D image of the external breast prosthesis is send to the additive manufacturing machine, for example an inkjet deposition printer wherein a molten polymer is deposited in the form of multiple separate droplets that fuse together after being deposited to become part of the printed structure.

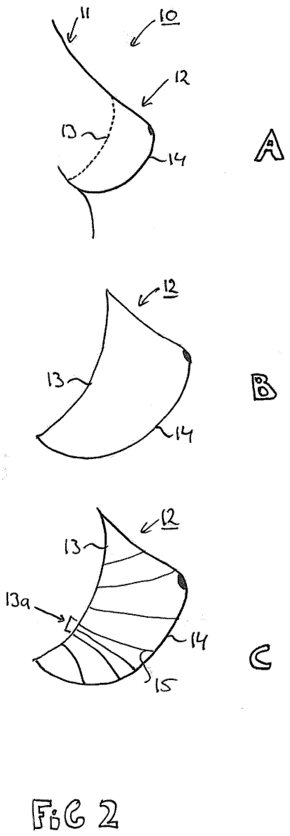

In Step 5 This machine manufactures a structure that corresponds to the 3D image of the breast prosthesis. This way, a unitary structure is made that can be used as an external breast prosthesis (for example in a brassiere), to arrive at the desired shape when positioned on the site of the operated breast. Thermal conductors can be dispersed in the structure during (or after) the printing process (see figure 2C). In case the thermal conductors are laid down on surfaces to be printed, they can be easily incorporated during the printing process. Alternatively, the conductors can be applied afterwards, for example by applying a sewing like process using a needle.

The method can be adapted to meet any individual desire for shape, feel, heat and moist conducting properties etc. depending on how the unitary structure is configured and depending on the use of any additional materials in the manufacturing process. For example, a structure according to the invention may be a reticulated solid foam, of which foam the porosity can be varied significantly, typically from 5 to 95%. This has a significant influence on the ultimate physical properties of the prosthesis. Also, the droplet size can be varied, as well as the polymer material used. Next to this, various additional materials can be added after or during the manufacture of the basic unitary structure. For example, a padding can be added if desired. Uisng all of the above and optionally other variations, the prosthesis can be completely individualized and even adapted to the occasion. For example, it is envisioned that for different levels of physical exercise, different prostheses are made for the same patient. Also, different prostheses may be made to fit different garments of the same patient.

### Figure 2

Figure 2, consisting of subfigures 2A, 2B and 2C schematically depicts an external breast prosthesis 12. In Figure 2A, the complete reconstructed breast 10 is depicted including the remaining breast tissue 11 after partial mastectomy, and the external prosthesis 12, including its inner surface that coincides with the tissue 11, and the opposing outer surface 14 that provides the outer visible shape of the reconstructed breast 10.

In Figure 2B only the external breast prosthesis 12 is depicted, the inner surface 13 and outer surface 14 being indicated as such. This is a unitary (single-piece) structure of a reticulated solid foam made of a thermoplastic elastomer, in this case CAWITON PR13620 (available from Wittenburg, Zeewolde, The Netherlands). The average porosity is 75%, ranging from 55% near the inner surface 13 to 85% near the outer surface 14.

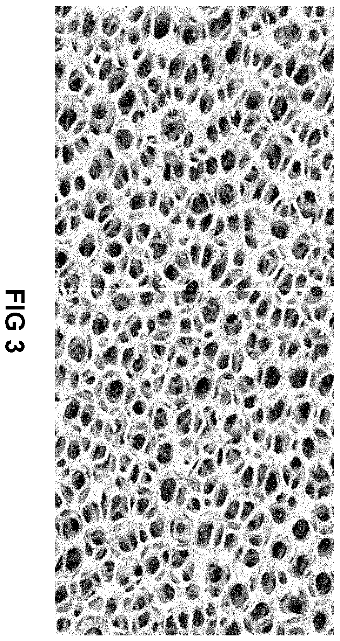

In Figure 3B the breast prosthesis is depicted with its multiple elongated thermal conductors 15 that each extend from a position adjacent the inner surface 13 of the breast prosthesis to a position adjacent to the outer surface 14 of the breast prosthesis to conduct heat away from the patient's body 11 towards surface 14. In this case, after having performed an IR scan of the body 11, it appeared that there was a small site of body 11, corresponding to section 13a of the inner surface, that produced significantly more heat than average, possibly an effect of the mastectomy operation. In order to make sure there is no significant local temperature deviation at this site, the density of the heat conductors is somewhat higher at site 13A. In the shown embodiment the elongated thermal conductors 15 are polymer fibres of DYNEEMA (available from DSM, Heerlen, The Netherlands) an oriented strand polyethylene that has a very good thermal conductivity (20 W/mK in axial direction). The conductors are provided in the prosthesis after the printing process of Step 5 (figure 1) by a sewing operation.

### Figure 3

Figure 3 schematically depicts a reticulated solid foam. The walls of the cells are made from the thermoplastic elastomer. As can be seen, the structure is porous and the cells are interconnected to form a so called open-cell-structured foam. Open-cell-structured foams contain pores that are connected to each other and form an interconnected network. Open-cell foams fill with whatever gas surrounds them. In the reticulated foam as depicted the pores are thus continuous, as opposed to local pores.

### Example 1

This example describes a method of manufacturing a breast prosthesis using an additive manufacturing technology. In this case the AM machine used was the ARBURG Freeformer 200-3X (Arburg, Lossburg, Germany), using CAWITON PR13640 (differing from PR1620 mainly in that the tensile moduli and tear strength are somewhat higher; the melting point is about the same, around 152°C) as the thermoplastic elastomer. Using a jet nozzle with a diameter of 0.2 mm, various structures were made by depositing individual droplets of the molten thermoplastic elastomer at an ejection temperature of 210°C towards a substrate to form a reticulate solid foam of fused droplets of the elastomer. A first reticulated solid foam structure was made by imposing 255 layers of droplets of 0.2 mm diameter to result in a prosthesis of about 248cm³, weighing 35 grams (corresponding to a porosity of about 85%). The total manufacturing process took about 20 hours.

The same way, various variants were made of the breast prosthesis, all reticulated solid foams of the same material, but differing in porosity from very high (95%) to low (25%). Table 1 gives an overview of the various structures.

**Table 1 Various reticulated solid foam TPE's**

| Prosthesis | Porosity |
|---|---|
| A | 95% |
| B | 85% |
| C | 75% |
| D | 50% |
| E | 25% |

### Example 2

This example describes various test performed on prostheses A through E as described in Example 1 to asses several bulk properties. In these prosthesis no thermal conductors were applied as no effect on the tested bulk properties of the prostheses were expected by the addition of a few polymer fibres in the prostheses. The first two tests pertain to the resilience of the prostheses after compression, and the third test pertains to the property to transport heat through the bulk material of the prostheses.

### Test 1

In this test the resilience after repeated compression and decompression is measured. For this a sample of each prosthesis A though E, having a thickness of about 19-20 mm, was subjected to 15 compression cycles (same force for every sample, leading to nearly full compression, i.e. 80-40% compression depending on porosity). After this, the sample height for each type of prosthesis was remeasured. As a positive control the height for a commercial silicon breast prosthesis sample was measured, which appeared to be 19.5 ± 0.4 mm. So any change at this level (0.4 mm) was found acceptable. The results are indicated in Table 2.

**Table 2 Sample height in mm after compression cycles**

| Prosthesis | 0 cycles | 15 cycles |
|---|---|---|
| A | 19.7 | 19.4 |
| B | 19.4 | 19.2 |
| C | 19.8 | 19.4 |
| D | 20.0 | 19.5 |
| E | 19.7 | 19.3 |

It can be seen that all types of prosthesis have an acceptable resiliency, albeit that at a porosity of 50% or lower there seems to be a tendency of less resilience (however still acceptable).

### Test 2

In this test the resilience after long term compression (1 cycle) is measured. Given the fact that all types of prosthesis appear to have almost the same resilience against repeated compression, the resilience against long term compression was only measured for prosthesis type C, having a porosity of 75%. The compression used was of the same level as that used in Test 1, but it was maintained for 24 hours. After this, the recovery was measured by measuring the sample height as a percentage of the original sample height before compression. A recovery to at least 90% in 3 hours was set as a threshold for adequate recovery after long term compression for an external breast prosthesis. The results are indicated in Table 3.

**Table 3 Recovery (%) after long term compression and X hours:minutes recovery time**

| Prosthesis | 0 h recovery | 1:13 h recovery | 2:12 h recovery | 15:36 h recovery |
|---|---|---|---|---|
| C | 54 | 92 | 95 | 96 |

It appeared that the recovery was acceptable, being already over 90% after 1 hour and 13 minutes recovery time.

### Test 3

The third test pertains to the property to transport heat through the bulk material of the prosthesis, based on mere unforced conduction through the prosthesis. For this samples of a common commercially available silicon breast prosthesis and prosthesis of type C (having the same dimensions) were heated one sided with a common air flow heat gun. After heating up the one side to a temperature 16°C above the opposing side, the samples were left and the difference in temperature of the two opposing sides was monitored for half an hour (1800 seconds). The results are indicated below in Table 4.

**Table 4 Temperature difference in °C after static heat transport for X sec.**

| Prosthesis | 0 | 200 | 400 | 800 | 1800 |
|---|---|---|---|---|---|
| C | 16 | 13 | 9 | 7 | 5 |
| Silicon | 16 | 15 | 16 | 15 | 13 |

It appeared that both types of prostheses leave a temperature difference, even after 1800 seconds. However, it also appeared that the bulk material of the prosthesis based on a reticulated solid foam of a TPE has a significantly increased capability of heat regulation when compared to common silicon breast prostheses. This may contribute significantly to mitigation of the problem of an external breast prosthesis getting uncomfortable by heat buildup at the surface where the chest and prosthesis coincide. For an even further improvement it is expected that by applying thermal conductors in the prostheses as tested (cf. Figure 2C) the temperature difference can be further decreased. When a conductor based on DYNEEMA fibres is used, it is expected that for both types of prostheses the temperature difference can be lowered to less than 1 or 2°C in 200-400 seconds given the very large heat conducting capacity of DYNEEMA fibres. If desired the heat regulation can be improved to any desired level by increasing the density of the fibres, i.e. the number of fibres per volume and/or the thickness of the fibres. The maximum density may depend on the resulting mechanical properties of the prosthesis, i.e. to what extent the properties still resemble those of natural breast tissue when the fibres are present at a large density.

## Claims

1. An external breast prosthesis (12) comprising a main body made from a polymer, the body having an inner surface (13) for coinciding with an outer chest surface (11) of a person wearing the breast prosthesis and an opposing outer surface (14), **characterised in that** the main body comprises multiple elongated thermal conductors (15) that each extend from a position adjacent the inner surface (13) of the breast prosthesis to a position adjacent the outer surface (14) of the breast prosthesis.

2. An external breast prosthesis according to claim 1, **characterised in that** the elongated thermal conductors (15) are fibres.

3. An external breast prosthesis according to claim 2, **characterised in that** the elongated thermal conductors (15) are polymer fibres.

4. An external breast prosthesis according to claim 3, **characterised in that** the elongated thermal conductors (15) are polymer fibres of an oriented strand polymer.

5. An external breast prothesis according to any of the preceding claims, **characterised in that** the main body consists essentially of a thermoplastic elastomer.

6. An external breast prosthesis according to claim 5, **characterised in that** the main body is a reticulated solid foam.

7. An external breast prosthesis according to claim 6, **characterised in that** the porosity of the reticulated solid foam is between 50 and 95%.

8. An external breast prosthesis according to claim 7, **characterised in that** the porosity of the reticulated solid foam is between 60 and 85%.

9. An external breast prosthesis according to any of the claims 5 to 7, **characterised in that** the thermoplastic elastomer is a styrene block copolymer.

10. An external breast prosthesis according to claim 9, **characterised in that** the thermoplastic elastomer is a styrene-ethylene-butylene-styrene copolymer.

11. An external breast prothesis according to any of the claims 5 to 9, **characterised in that** the thermoplastic elastomer is deposited in the form of multiple separate droplets that are fused together.

12. An external breast prosthesis according to claim 11, **characterised in that** the droplets are deposited via a 3D printing process.

13. An external breast prosthesis according to claim 12, **characterised in that** the droplets are deposited via an ink droplet deposition process.

## Patentansprüche

1. Externe Brustprothese (12), umfassend einen aus einem Polymer hergestellten Hauptkörper, wobei der Körper eine innere Oberfläche (13) zum Zusammentreffen mit einer äußeren Brustoberfläche (11) einer Person, die die Brustprothese trägt, und eine gegenüberliegende äußere Oberfläche (14) aufweist, **dadurch gekennzeichnet, dass** der Hauptkörper mehrere längliche Wärmeleiter (15) umfasst, die sich jeweils von einer der inneren Oberfläche (13) der Brustprothese benachbarten Position zu einer der äußeren Oberfläche (14) der Brustprothese benachbarten Position erstrecken.

2. Externe Brustprothese nach Anspruch 1, **dadurch gekennzeichnet, dass** die länglichen Wärmeleiter (15) Fasern sind.

3. Externe Brustprothese nach Anspruch 2, **dadurch gekennzeichnet, dass** die länglichen Wärmeleiter (15) Polymerfasern sind.

4. Externe Brustprothese nach Anspruch 3, **dadurch gekennzeichnet, dass** die länglichen Wärmeleiter (15) Polymerfasern aus einem orientierten Strangpolymer sind.

5. Externe Brustprothese nach einem der vorhergehenden Ansprüche, **dadurch gekennzeichnet, dass** der Hauptkörper im Wesentlichen aus einem thermoplastischen Elastomer besteht.

6. Externe Brustprothese nach Anspruch 5, **dadurch gekennzeichnet, dass** der Hauptkörper ein netzartiger fester Schaumstoff ist.

7. Externe Brustprothese nach Anspruch 6, **dadurch gekennzeichnet, dass** die Porosität des netzartigen festen Schaums zwischen 50 und 95% liegt.

8. Externe Brustprothese nach Anspruch 7, **dadurch gekennzeichnet, dass** die Porosität des netzartigen festen Schaums zwischen 60 und 85% liegt.

9. Externe Brustprothese nach einem der Ansprüche 5 bis 7, **dadurch gekennzeichnet, dass** das thermoplastische Elastomer ein Styrolblockcopolymer ist.

10. Externe Brustprothese nach Anspruch 9, **dadurch gekennzeichnet, dass** das thermoplastische Elastomer ein Styrol-Ethylen-Butylen-Styrol-Copolymer ist.

11. Externe Brustprothese nach einem der Ansprüche 5 bis 9, **dadurch gekennzeichnet, dass** das thermoplastische Elastomer in Form von mehreren separaten Tröpfchen, die miteinander verschmolzen sind, aufgetragen wird.

12. Externe Brustprothese nach Anspruch 11, **dadurch gekennzeichnet, dass** die Tröpfchen durch ein 3D-Druckprozess aufgebracht werden.

13. Externe Brustprothese nach Anspruch 12, **dadurch gekennzeichnet, dass** die Tröpfchen durch einen Tintentröpfchen-Auftragsprozess aufgebracht werden.

## Revendications

1. Prothèse mammaire externe (12)
comprenant un corps principal en polymère, le corps présentant une surface interne (13) destinée à venir en contact avec la surface externe de la poitrine (11) d'une personne portant la prothèse mammaire, ainsi qu'une surface extérieure opposée (14), **caractérisée en ce que** le corps principal comprend plusieurs conducteurs thermiques allongés (15), chacun s'étendant depuis une position adjacente à la surface interne (13) de la prothèse mammaire jusqu'à une position adjacente à la surface externe (14) de la prothèse mammaire.

2. Prothèse mammaire externe selon la revendication 1, **caractérisée en ce que** les conducteurs thermiques allongés (15) sont des fibres.

3. Prothèse mammaire externe selon la revendication 2, **caractérisée en ce que** les conducteurs thermiques allongés (15) sont des fibres polymères.

4. Prothèse mammaire externe selon la revendication 3, **caractérisée en ce que** les conducteurs thermiques allongés (15) sont des fibres polymères d'un polymère à chaînes orientées.

5. Prothèse mammaire externe selon l'une des revendications précédentes, **caractérisée en ce que** le corps principal est constitué essentiellement d'un élastomère thermoplastique.

6. Prothèse mammaire externe selon la revendication 5, **caractérisée en ce que** le corps principal est une mousse solide réticulée.

7. Prothèse mammaire externe selon la revendication 6, **caractérisée en ce que** la porosité de la mousse solide réticulée est comprise entre 50 et 95 %.

8. Prothèse mammaire externe selon la revendication 7, **caractérisée en ce que** la porosité de la mousse solide réticulée est comprise entre 60 et 85 %.

9. Prothèse mammaire externe selon l'une des revendications 5 à 7, **caractérisée en ce que** l'élastomère thermoplastique est un copolymère à blocs de styrène.

10. Prothèse mammaire externe selon la revendication 9, **caractérisée en ce que** l'élastomère thermoplastique est un copolymère styrène-éthylène-butylène-styrène.

11. Prothèse mammaire externe selon l'une des revendications 5 à 9, **caractérisée en ce que** l'élastomère thermoplastique est déposé sous la forme de multiples gouttelettes distinctes fusionnées entre elles.

12. Prothèse mammaire externe selon la revendication 11, **caractérisée en ce que** les gouttelettes sont déposées au moyen d'un procédé d'impression 3D.

13. Prothèse mammaire externe selon la revendication 12, **caractérisée en ce que** les gouttelettes sont déposées au moyen d'un procédé de dépôt de gouttelettes d'encre.
